Europäisches Patentamt

European Patent Office

Office européen des brevets

(11) Publication number: **0 436 852 A1**

## (12) EUROPEAN PATENT APPLICATION

(21) Application number: **90123811.3**

(22) Date of filing: **11.12.90**

(51) Int. Cl.⁵: **A61B 19/08**

(30) Priority: **14.12.89 US 450876**

(43) Date of publication of application:
**17.07.91 Bulletin 91/29**

(84) Designated Contracting States:
**AT BE DE DK FR GB**

(71) Applicant: **KIMBERLY-CLARK CORPORATION**
**401 North Lake Street**
**Neenah Wisconsin 54956(US)**

(72) Inventor: **Grier-Idris, Carletta**
**1362 Meadows Creek Way, N.W.**
**Acworth, Georgia 30101(US)**
Inventor: **Wilson, Patrick Clark**
**6216 Rosecommon Drive**
**Norcross, Georgia 30092(US)**

(74) Representative: **Diehl, Hermann Dr. et al**
**Diehl & Glaeser, Hiltl & Partner**
**Flüggenstrasse 13**
**W-8000 München 19(DE)**

(54) **Two layer fenestrated incise system.**

(57) Disclosed herein are a two layer fenestrated incise system (10) for surgical drapes (11) and the resultant surgical drape (11). The incise system (10) is a two layer structure including a first layer (12) of incise with an adhesive bottom surface for adhering the layer (12) to a surgical drape (11) and/or the skin of a patient. The second layer (14) of incise is generally coetensive with and releasably attachable to the first layer (12) of incise so as to cover the fenestration (26) of the first layer.

To use the incise system of the present invention the two layer structure is applied to the patient's skin such that the fenestration (26) of the first layer (12) of incise directly overlies the intended incision area. An incision can then be made directly through the two layers (12, 14) of incise in the area of the fenestration in the first layer. Once the procedure is completed, the top (second) layer (14) of incise is peeled from the bottom (first) layer (12). As the top layer (14) is removed, the fenestration area (26) of the first layer is revealed thereby exposing a portion of the patient's skin surrounding the incision. As a result, suturing of the incision is make easier since it is no longer necessary to pick and peel the incise away from the incision to expose sufficient skin to allow suturing without catching the incise in the process.

FIG. 3

## TWO LAYER FENESTRATED INCISE SYSTEM

The present invention relates to an incise material suitable for use in surgical procedures.

Many of today's surgical procedures involve the use of an incise material. An incise material is usually a clear polymeric film with an adhesive on one side which is in turn covered with a release paper. Two suppliers of incise material are the Minnesota Mining and Manufacturing Company and T. J. Smith and Nephew Ltd. Examples of incise material can be found, by way of example only, in U.S. Patent Nos. 4,310,509; 4,323,557; 4,452,845; RE. 31,886 and RE. 31,887. Most typically incise material is used in connection with towels or surgical drapes to maintain the surgical area as clean and sterile as possible to help reduce the risk of postoperative infection. Once the surgical area of the patient has been scrubbed and treated with a bacteriostat, the surgical site is squared-off by the use of sterile towels or a surgical drape which has a fenestration of a size which is larger than the expected size of the incision. An incise material is then used to cover all or a portion of the patient's skin left exposed by the towels or the fenestration in the surgical drape. Some surgeons prefer to use incise materials which themselves have fenestrations which are only slightly larger than the incision area. Other surgeons prefer to use incise materials which completely cover the incision area with the incision being made directly through the incise material. In either case, to apply the incise the releasable backing is removed and the adhesive side of the material is applied directly to the skin of the patient. One purpose in using the incise material is to help reduce the migration of germs and bacteria to the incision site. This is because, despite the cleansing of the skin, the pores still contain additional germs and bacteria which can migrate to the surface as the skin is moved and worked during the course of the surgical procedure. By covering the skin, it has been found that this migration can be reduced.

When an incise is used which completely covers the incision area the surgeon will cut right through the incise material attached to the skin. In this manner the amount of exposed skin surrounding the incision is minimized. A problem arises, however, when it comes time to close the incision. At this point the incise material directly surrounding the incision must be peeled back so as to expose sufficient skin for the suturing procedure. This process of peeling the incise back is, at the very least, a time consuming and annoying process. Because of this problem, some surgeons elect to use the above-described fenestrated incise material to eliminate the need for peeling back the incise material. This, however, is often a compromise decision since, in using a fenestrated incise material, more skin is exposed about the incision site thereby increasing the risk of bacteria and germs making their way to the incision and causing infection.

It is therefore an object of the present invention to provide an incise system which will allow the surgeon to cut directly through the incise material while at the same time permitting easy exposure of the skin about the incision when it comes time to close the incision.

The invention solves this object by providing an incise system according to independent claim 1 and a surgical drape containing same according to independent claim 18 and 23. Further advantageous features of the incise system and the drape are evident from the dependent claims, the description and the drawings. The claims are intended to be understood as a first non-limiting approach of defining the invention in general terms.

The invention provides an incise material which is of a two layer structure which has a removable top layer to facilitate suturing of an incision upon completion of a surgical procedure.

The above and other aspects of the present invention will become more apparent upon a further review of the following specification and drawings.

Figure 1 is a top plan view of an incise system according to the present invention.

Figure 2A is a perspective view of the first or bottom layer of an incise system according to the present invention.

Figure 2B is a side view of the material shown in Figure 2A.

Figure 2C is a perspective view of the second or top layer of an incise system according to the present invention.

Figure 2D is a side view of the material shown in Figure 2C.

Figure 2E is a side view of an incise system according to the present invention.

Figure 3 is a top plan view of an incise system according to the present invention attached to a surgical drape with the second layer of incise being separated from the first layer.

Figure 4 is a cross-sectional view of Figure 3 taken along line 4-4.

Figure 5 is a side view of the first and second layers of incise with separate release liners according to the present invention.

Figure 6 is a side view of a first and second layer of incise when joined along a common edge according to the present invention.

Figure 7 is another perspective view of an incise system according to the present invention.

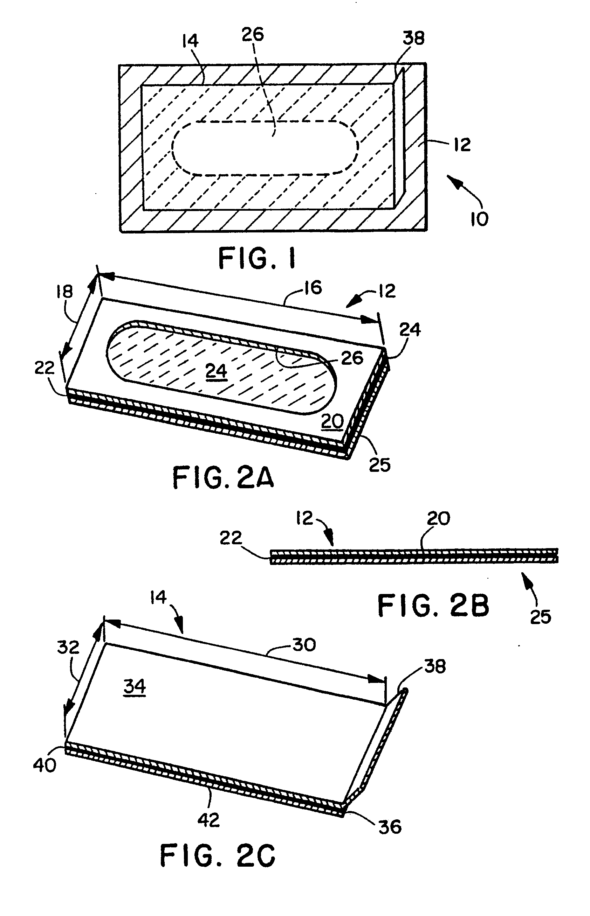

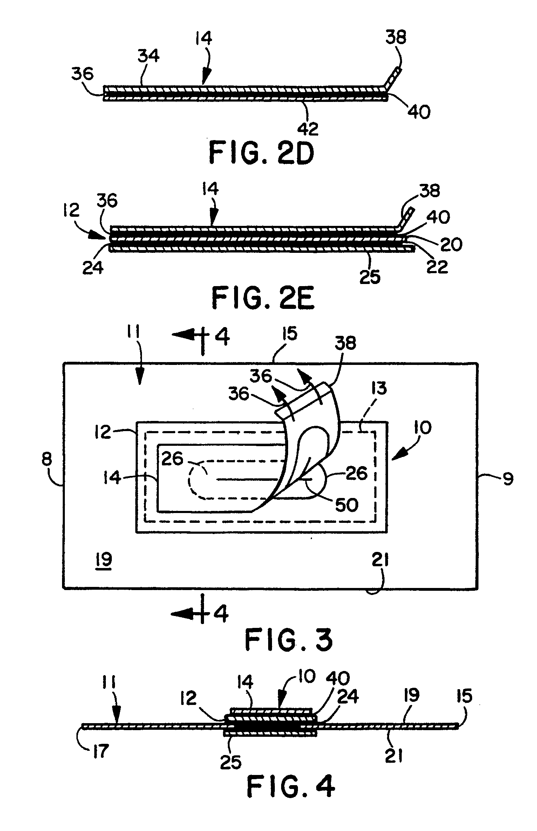

Referring to Figures 1 through 7 there is shown an incise system 10 according to the present invention. The incise system 10 is comprised of a first layer 12 and a second layer 14 of incise. Such incise is readily available and well known to those having ordinary skill in the art. Examples of an incise including adhesives suitable for the present invention include Opsite® Incise 63110; 63210; 63310 and 63410 from T. J. Smith and Nephew Inc., 2000 South Beltline Blvd., Columbia, South Carolina, U.S.A. 29205. The incise system 10 including the first layer 12 and second layer 14 may be manufactured and sold as a separate unit or it may be sold as part of a surgical pack in which case the system 10 may be unattached or pre-attached to a surgical drape or mainsheet 11 having a primary fenestration 13, a top edge 8 and a bottom edge 9 joined by a pair of opposed side edges 15 and 17. The mainsheet 11 further includes a top surface 19 and a bottom surface 21. See Figure 3. In any event, the first layer 12 should be of a size sufficient to cover all sides of the fenestration 13 in the surgical drape 11.

Referring to Figures 2A and 2B, the first layer 12 is a continuous layer of incise having a first length 16 and a first width 18. The first layer 12 has a top surface 20 and a bottom surface 22. Affixed to the bottom surface 22 is a first adhesive layer 24, the composition of which is well known to those having ordinary skill in the art. Attached to the first adhesive layer 24 is a release paper 25, the composition of which is also well known to those having ordinary skill in the art. Additionally, the first layer 12 has a fenestration 26 located therein. The fenestration area 26 should generally be at least an inch * longer at either end than the intended length of the incision. The width of the fenestration area 26 should also generally be at least an inch wider on either side of the incision. This will assure that their is sufficient skin exposed to permit suturing.

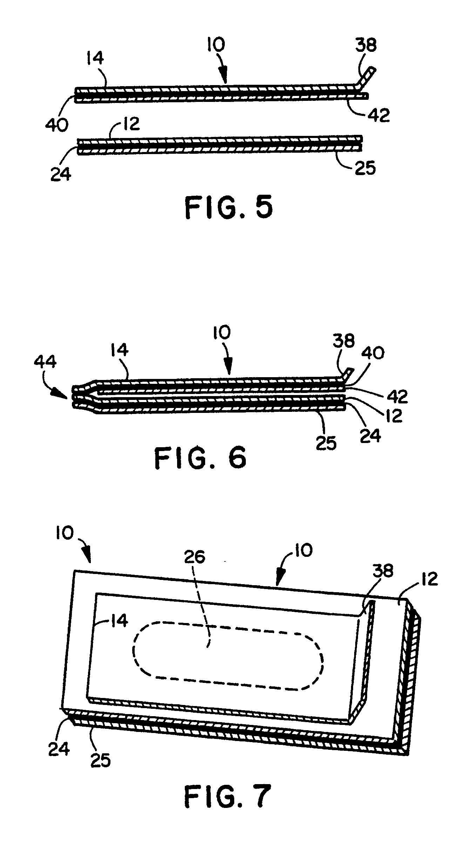

Referring to Figures 2C and 2D, the second layer of incise 14 is a continuous sheet having a second length 30 and a second width 32 which are generally coextensive with the first length 16 and first width 18 of the first layer 12. Layer 14 also has a top surface 34 and a bottom surface 36. As shown in Figures 1 and 7, first layer 12 is slightly larger than the second layer 14, however, the first layer 12 also may be smaller than or equal in size to the second layer 14 so long as the second layer 14 covers the fenestrated area 26 of the first layer 12. To facilitate removal of the second layer 14 from the first layer 12, the second layer 14 may be provided with one or more pull tabs 38 located along the edges of the second layer 14. In its simplest form the pull tab 38 may be a section of the second layer 14 folded over and adhered to itself via an adhesive. Alternatively, the pull tab 38 may be a separate element such as an adhesive-coated paper with instructions or other indicia located thereon and affixed to one of the ends of the second layer 14. The tab 38 should be of sufficient size to permit adequate grasping and pulling of the tab 38 when releasing the second layer 14 from the first layer 12. Generally, a height of one inch or more should suffice.

To releasably adhere the first layer 12 and the second layer 14 together, a second adhesive layer 40 similar to the previously mentioned adhesive layer 24 is positioned between the two layers 12 and 14. This second adhesive layer 40 may be applied to the top surface 20 of the first layer 12 or to the bottom surface 36 of the second layer 14. In Figure 2D the adhesive layer 40 is shown attached to the bottom surface 36 of the second layer 14. Generally, the two layers 12 and 14 are adhered to one another during the manufacturing of the present invention so that the two layers may be applied together to the patient's skin thereby lessening the chance of wrinkling. See Figure 2E. Alternatively, however, as shown in Figure 2D a release liner 42 may be attached to one side of the exposed second adhesive layer 40 to keep the two layers 12 and 14 separated until attachment of the two layers is desired. In such a configuration, the first and second layers 12 and 14 may be completely separated as shown in Figure 5. On the other hand, the two layers 12 and 14 may be joined along a common edge 44 as shown in Figure 6. In addition, this common edge 44 may be attached to the top surface 19 of the mainsheet 11 adjacent the fenestration 13. Most commonly this edge 44 is opposite the pull tab 38. Furthermore, the release paper 42 should not extend completely across the juxtaposed bottom surface 36 of second layer 14 and the top surface 20 of the bottom layer 12. Instead, the release paper 42 should extend from the end of the layers 12 and 14 adjacent the pull tab 38 to a point adjacent the joined common edge 44. In this manner the adhesive 40 can be used to adhere and hinge the two layers 12 and 14 together. See Figure 6.

As stated at the outset, the incise system 10 of the present invention may be either unattached to the drape (as shown in Figures 1, 5, 6 and 7) or preattached (as shown in Figures 3 and 4). When the incise system is attached to the drape 11, it should be large enough to cover the fenestration 13 in the drape 11 as shown in Figures 3 and 4. Also, in most cases the release paper 25 will be attached to the underside of the drape 11 as shown

* 1 inch = 2.54 cm

in cross-section in Figure 4 via the adhesive layers 24 and 40.

Having thus described several embodiments of the incise system 10, the application and removal of the system will be explained.

When the incise system 10 of the present invention is preattached to a surgical drape as is the case in Figures 3 and 4, the drape 11 is applied to the patient in the same manner as would normally be prescribed for the specific type of drape. To apply the incise, the release paper 25 is removed thereby exposing the adhesive 24 for adhesion to the patient's skin in the intended area of the surgical procedure. The only added precaution is that the fenestrated area 26 be properly aligned so that the incision 50 is made within the confines of the fenestrated area 26. Once the drape 11 and incise system 10 are in place, an incision 50 can be made through layer 12, the fenestrated area 26 of layer 14 and the patient's skin within the area defined by fenestrated area 26. Both the skin and incise can then be pulled back and clamped to facilitate the surgical procedure.

To use the incise system shown in Figure 5 which employs two release papers 25 and 42, the two layers are applied in sequence with the first layer 12 being first applied to the skin of the patient (not shown) followed by the application of layer 14. As with the first embodiment, once the system has been applied, an incision is made in the skin through the two layers of incise 12 and 14 in the area of the fenestration area 26 of layer 12. Both the skin and the incise can then be pulled back and clamped to facilitate the surgical procedure.

To use the incise system 10 shown in Figures 2E and 7, the release paper 25 is removed from the bottom 22 of the system 10 thereby exposing the adhesive 24 on the bottom of the first layer 12. The adhesive side is then applied to the skin (not shown) of the patient and an incision is made through the skin and the area of the second layer 14 which directly overlies the fenestration area 26 in the first layer 12. Both the skin and the incise can then be pulled back and clamped to facilitate the surgical procedure.

To use the incise system 10 shown in Figure 6, the release paper 25 is removed from the bottom 22 of the system 10 thereby exposing the adhesive 24 on the bottom of the first layer 12. The adhesive 24 is applied to the patient's skin such that the fenestrated area 26 directly overlies the intended site of the incision. Next, the release liner 42 is removed from the second layer of incise 14 thereby exposing the adhesive 40 for adhesion of the second layer 14 to the top of the first layer 12. As with the other embodiments, once the system 10 has been applied, an incision is made through the skin (not shown) and the two layers of incise 12 and 14 in the area of the fenestration area 26 of layer 12. Both the skin and the incise can then be pulled back and clamped to facilitate the surgical procedure.

Once the procedure has progressed to the point of closure, the top or second layer 14 is removed by grasping and pulling the tab 38 in the direction of arrows 36 as shown in Figure 3. As the second layer is pulled/peeled back, the fenestrated area 26 of first layer 12 is revealed thereby providing an area of exposed skin about the incision to permit suturing without interference from the incise material. When separating the top or second layer 14 from the bottom or first layer 12, it is desirable to have the first layer maintain contact with the skin of the patient. Consequently, when using the same incise material and adhesive for both layers, it may be necessary to hold the first layer 12 down with one hand while pulling on the tab 38 of the second layer 14. In a preferred embodiment, the adhesive layer 24 used to adhere the incise system 10 to the patient's skin via the first layer 12 should be stronger than the adhesive 40 used to adhere the second layer 14 to the first 12. As a result, in a preferred embodiment, the first layer 12 can be said to have a first peel strength and the second layer 14 can be said to have a second peel strength. To maintain a higher degree of adhesion of the first layer 12 to the patient's skin as compared to the adhes ion of the second layer 14 to the first 12, the first peel strength should be greater than the second peel strength. One standard that can be used to measure such peel forces/strengths is PSTC-1 (November 1970 revised version of the September 1955 test) developed by the Pressure Sensative Tape Council, 1201 Waukegan Road, Glenview, Illinois 60025. Finally, having completed the suturing, the first layer 12 of incise may be removed from the patient, most commonly in conjunction with the removal of the mainsheet 11.

Having thus described the invention in detail it should be appreciated that various other modifications can be made without departing from the spirit and scope of the appended claims. For example, the fenestrated area 26 instead of being located entirely within the incise may extend over to one edge of the incise to create a "U-shaped" or other cut-out portion for limb surgery.

## Claims

1. An incise system (10) for use in surgical procedures comprising:
   a first layer (12) of incise material having a top surface (20) and a bottom surface (22), said first layer (12) defining a fenestration (26) therein, and

a second layer (14) of incise releasably attachable to said top surface (20) of said first layer (12), said second layer (14) having a top surface (34) and a bottom surface (36) sufficient to cover said fenestration (26) in said first layer (12).

2. The incise system of claim 1 wherein said first layer (12) includes an adhesive (24) on said bottom surface (22) of said first layer whereby said first layer has a first peel strength and said second layer (14) of incise includes an adhesive (40) on said bottom surface of said second layer whereby said second layer (14) has a second peel strength, said first peel strength being greater than said second peel strength.

3. The incise system of claim 1 or 2 which further includes a release liner (25) releasably attached to said bottom surface (22) of said first layer (12) of incise.

4. The incise system of one of the preceding claims, wherein said second layer (14) of incise further includes a release liner (42) releasably attached to said bottom surface (36) of said second layer of incise.

5. The incise system of one of the preceding claims, wherein said second layer (14) of incise has a pull tab (38) attached thereto to facilitate removal of said second layer (14) of incise from said first layer (12) of incise.

6. The incise system of one of the preceding claims, wherein said first (12) and second (14) layer of incise are attached to one another along a common edge (44).

7. The incise system of one of the preceding claims, in which the first layer (12) of incise has a first length (16) and a first width (18) and the second layer (14) of incise has a second length (30) and a second width (32), said second length (30) and width (32) being sufficient to cover said fenestration (26) in said first layer.

8. The incise system of one of the preceding claims, in which said second layer (14) of incise covers the full top surface (20) of said first layer (12) of incise.

9. The incise system of one of the preceding claims further comprising a main sheet (11) having a primary fenestration (13), the first layer (12) of incise being arranged such that at least part of its fenestration (26) and preferably all of same is arranged within the boundaries of said primary fenestration (13) of said mainsheet (11).

10. The incise system of claim 9, in which said first layer (12) of incise is arranged such that the borderline of its fenestration (26) is spaced from the borderline of said primary fenestration (13) of said mainsheet.

11. The incise system of claim 9 or 10, in which said first layer (12) of incise is arranged on the top side (19) of said mainsheet (11).

12. The incise system of one of claims 9 to 11, in which said second layer (14) of incise is attachable to said top surface (20) of said first layer (12) of incise and/or said first layer (12) of incise and said second layer (14) of incise are attached to one another along a common edge (44) with said common edge being attached to said top surface of said mainsheet (11) adjacent said primary fenetration (13).

13. The incise system of one of claims 9 to 12 which further includes a release liner (25) located adjacent a bottom surface (21) of said main sheet (11) and releasably attached to said bottom surface (22) of said first layer (12) of incise and/or said bottom surface (21) of said main sheet.

14. The incise system of claim 9 or 10, in which at least said first layer (12) of incise is arranged below the bottom surface (21) of said main sheet (11).

15. The incise system of claim 14, in which said first layer (12) of incise is attached via its top surface (20) to the bottom surface (21) of said mainsheet.

16. The incise system of claim 14 or 15, in which said second layer (14) of incise is arranged completely within said primary fenestration (13) of said mainsheet (11).

17. The incise system of one of claims 14 to 16, in which said second layer (14) of incise is releasably attached to said top surface (20) of said first layer (12) of incise and/or the top surface (19) of said main sheet. (11).

18. A surgical drape comprising:
     a mainsheet (11) having a top surface (19) and a bottom surface (21), said mainsheet (11) defining a primary fenestration (13) therein, said mainsheet (11) further including a two layer incise system (10) for covering said primary fenestration (13) with said system (10) including a first (12) and second (14) layer of incise,

said first layer (12) of incise having a top surface (20), and a bottom surface (22) for adhering said first layer (12) to said mainsheet (11) to cover said primary fenestration (13) and to the skin of a person, said first layer (12) further defining a fenestration (26) therein,

said second layer (14) of incise being releasably attachable to said top surface (20) of said first layer (12), said second layer (14) having a size sufficient to cover said fenestration (26) in said first layer (12).

19. The surgical drape of claim 18 wherein said first layer (12) of incise includes an adhesive (24) on said bottom surface of said first layer whereby said first layer has a first peel strength and said second layer (14) of incise includes an adhesive (40) on said bottom surface of said second layer whereby said second layer has a second peel strength, said first peel strength being greater than said second peel strength.

20. The surgical drape of claim 18 or 19, which further includes a release liner (25) located adjacent said bottom surface of said mainsheet (11) and releasably attached to said bottom surface (22) of said first layer (12) of said incise in the area of said primary fenestration (13) of said mainsheet (11).

21. The surgical drape of one of claims 18 to 20, wherein said second layer (14) of incise has a pull tab (38) attached thereto to facilitate removal of said second layer (14) of incise from said first layer (12) of incise.

22. The surgical drape of one of claims 18 to 21, wherein said first (12) and second (14) layers of incise are attached to one another along a common edge (44) with said common edge being attached to said top surface of said mainsheet (11) adjacent said primary fenestration (13).

23. A surgical drape comprising:
a mainsheet (11) having a top surface and a bottom surface, said mainsheet also defining a primary fenestration (13) therein, and a two layer incise system (10) for covering said primary fenestration (13) including a first layer (12) and a second layer (14) of incise joined along a common edge (44), said first layer (12) of incise having a top surface, and a bottom surface for adhering said first layer (12) of incise to said mainsheet (11) to cover said primary fenestration (13) of said mainsheet, said first layer further defining a fenestration (26) therein,

said second layer (14) of incise being releasably attachable to said top surface of said first layer (12), said second layer having a size sufficient to cover said fenestration (26) in said first layer (12), said second layer (14) having a pull tab (38) attached thereto along an edge opposite to said common edge (44) to facilitate separation of said second layer (14) of incise from said first (12) layer, of incise.

24. The surgical drape of claim 23, wherein said first layer (12) and said second layer (14) of incise each having a release liner (25; 40) removably attached thereto.

25. The surgical drape of claim 23 or 24, wherein said first layer (12) of incise includes an adhesive (24) on said bottom surface of said first layer whereby said first layer has a first peel strength and said second layer (14) of incise includes an adhesive (40) on said bottom surface of said second layer whereby said second layer has a second peel strength, said first peel strength being greater than said second peel strength.

26. The surgical drape of one of claims 18 to 25, in which the mainsheet (11) has a top edge (8) and a bottom edge (9) joined by a pair of opposed side edges (15, 17), the first layer (12) of incise has a first length (16) and a first width (18) and the second layer (14) of incise has a second length (30) and a second width (32) sufficient to cover said fenestration (26) in said first layer (12).

FIG. 1

FIG. 2A

FIG. 2B

FIG. 2C

FIG. 2D

FIG. 2E

FIG. 3

FIG. 4

FIG. 5

FIG. 6

FIG. 7

European
Patent Office

EUROPEAN SEARCH
REPORT

Application Number

EP 90 12 3811

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| Y | EP-A-0 166 124 (SURGIKOS) <br> * Page 2, line 29 - page 4, line 5; figures 1-4 * | 1-7,9-21, 26 | A 61 B <br> 19/08 |
| Y | US-A-4 024 862 (R.F. COLLINS) <br> * The abstract * | 1-7,9-21, 26 | |
| Y | GB-A-2 120 104 (KENDALL) <br> * The abstract; page 2, lines 2-5; figure 4 * | 2,21,26 | |
| Y | FR-A-2 581 545 (MINNESOTA) <br> * The abstract * | 4-6 | |
| Y | US-A-4 730 609 (B.E. McCONNELL) <br> * Column 4, lines 18-34; figures 2,3 * | 14,15 | |
| A | US-A-4 489 720 (H.K. MORRIS et al.) <br> * Column 3, lines 5-17; figures 1,4 * | 1,3 | |
| A | US-A-4 641 643 (L.H. GREER) <br> * The whole document * | 1-3 | |

| | | | TECHNICAL FIELDS SEARCHED (Int. Cl.5) |
|---|---|---|---|
| | | | A 61 B <br> A 61 F <br> A 61 M |

The present search report has been drawn up for all claims

| Place of search | Date of completion of search | Examiner |
|---|---|---|
| The Hague | 14 March 91 | WOLF C.H.S. |